# EUROPÄISCHE PATENTSCHRIFT

(11) **EP 0 470 463 B1**
(45) Veröffentlichungstag und Bekanntmachung des Hinweises auf die Patenterteilung: **26.02.1997**
(21) Anmeldenummer: 91112636.5
(22) Anmeldetag: 27.07.1991
(51) Int. Cl.: C07D 521/00, A01N 43/653, A01N 43/50

(54) **Halogenalkyl-azolyl-Derivate**
Alkyl-halide-azolyl derivatives
Dérivés d'azolyle halogénalkylé

(30) Priorität: 09.08.1990 DE 4025204; 10.01.1991 DE 4100515
(43) Veröffentlichungstag der Anmeldung: 12.02.1992
(73) Patentinhaber: BAYER AG, 51368 Leverkusen (DE)
(72) Erfinder: Jautelat, Manfred, Dr., W-5093 Burscheid (DE); Scherkenbeck, Jürgen, Dr., W-5090 Leverkusen 3 (DE); Stroech, Klaus, Dr., W-5650 Solingen 19 (DE); Dutzmann, Stefan, Dr., W-4010 Hilden 1 (DE); Dehne, Heinz-Wilhelm, Dr., W-4019 Monheim (DE); Hänssler, Gerd, Dr., W-5090 Leverkusen 3 (DE); Kuck, Karl-Heinz, Dr., W-4018 Langenfeld (DE)

(56) Entgegenhaltungen:
- EP-A- 0 223 327
- EP-A- 0 318 400

## Beschreibung

Die vorliegende Erfindung betrifft neue Halogenalkyl-azolyl-Derivate, mehrere Verfahren zu deren Herstellung und deren Verwendung als Mikrobizide im Pflanzenschutz und im Materialschutz.

Es ist bekannt geworden, daß bestimmte Dihalogen-allyl-triazolyl-Derivate fungizide Eigenschaften besitzen (vgl. EP-A 0 097 425). So lassen sich z.B. 4-(2,4-Dichlor-phenyl)-1,2-dibrom-4-hydroxy-5-(1,2,4-triazol- 1 -yl)-pent- 1 -en und 4-(2,4-Dichlor-phenyl)-1,2-dichlor-4-hydroxy-5-(1,2,4-triazol-1-yl)-pent-1-en zur Bekämpfung von Pilzen einsetzen. Die Wirkung dieser Stoffe ist gut, läßt aber bei niedrigen Aufwandmengen in manchen Fällen zu wünschen übrig.

Weiterhin ist schon bekannt, daß bestimmte Halogenalkyl-triazolyl-Derivate, die am zentralen Kohlenstoffatom einen gegebenenfalls substituierten Phenylrest enthalten, als Fungizide gegen phytopathogene Pilze geeignet sind (vgl. EP-A 0 380 277). Entsprechende Verbindungen, in denen die gegebenenfalls substituierte Phenylgruppe durch einen anderen Rest ersetzt ist, werden jedoch nicht erwähnt.

Außerdem geht aus der EP-A 0 318 400 hervor, daß Hydroxyethyl-azolyl-Derivate, die am Carbinol-Kohlenstoffatom einen Halogenpropyl-Rest und einen gegebenenfalls substituierten Phenyl- beziehungsweise Benzyl-Rest tragen, fungizid wirksam sind. Es werden aber keine Verbindungen dieses Typs offenbart, die statt des Phenyl- bzw. Benzyl-Substituenten einen gegebenenfalls durch Halogen oder Cycloalkyl substituierten Alkylrest oder einen gegebenenfalls substituierten Cycloalkyl-Rest enthalten.

Schließlich sind aus der EP-A 0 223 327 Triazolyl-Derivate mit pflanzenwuchsregulierenden Eigenschaften bekannt, in denen das zentrale Kohlenstoffatom sowohl mit gegebenenfalls halogeniertem tert.-Butyl als auch mit einem weiteren Halogenalkyl-Rest verknüpft sein kann. Allerdings werden keine Verbindungen mit einem tetrahalogenierten Alkylrest erwähnt.

Es wurden nun neue Halogenalkyl-azolyl-Derivate der Formel 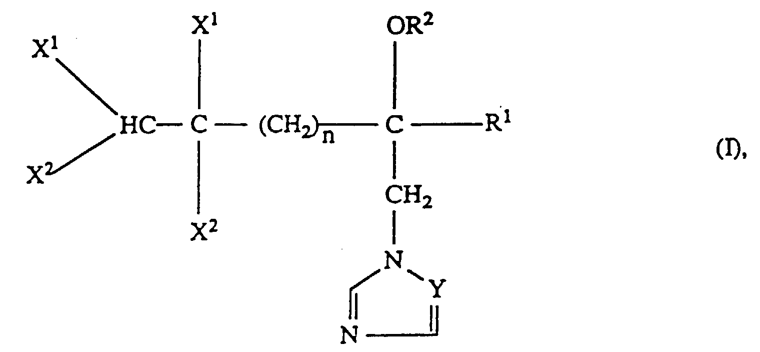 in welcher
- R¹: für geradkettiges oder verzweigtes Alkyl mit 1 bis 6 Kohlenstoffatomen steht, wobei jeder dieser Reste einfach bis dreifach, gleichartig oder verschieden substituiert sein kann durch Halogen und/oder Cycloalkyl mit 3 bis 7 Kohlenstoffatomen oder
- R¹: für Cycloalkyl mit 3 bis 7 Kohlenstoffatomen steht, wobei jeder dieser Cycloalkylreste einfach bis dreifach, gleichartig oder verschieden substituiert sein kann durch Halogen und/oder Alkyl mit 1 bis 4 Kohlenstoffatomen,
- R²: für Wasserstoff, Alkyl mit 1 bis 6 Kohlenstoffatomen, Alkenyl mit 3 bis 6 Kohlenstoffatomen, Acyl mit 1 bis 4 Kohlenstoffatomen oder Phenylalkyl mit 1 bis 4 Kohlenstoffatomen im Alkylteil steht,
- X¹: für Fluor, Chlor oder Brom steht,
- X²: für Fluor, Chlor oder Brom steht,
- n: für 0 oder 1 steht und
- Y: für Stickstoff oder eine CH-Gruppe steht,
sowie deren Säureadditions-Salze und Metallsalz-Komplexe gefunden.

Die Verbindungen der Formel (I) enthalten mindestens ein asymmetrisch substituiertes Kohlenstoffatom und können deshalb in optischen Isomerenformen anfallen. Die vorliegende Erfindung betrifft sowohl die Isomerengemische als auch die einzelnen Isomeren.

Weiterhin wurde gefunden, daß man Halogenalkyl-azolyl-Derivate der Formel (I) sowie deren Säureadditions-Salze und Metallsalzkomplexe erhält, wenn man

Alkine der Formel 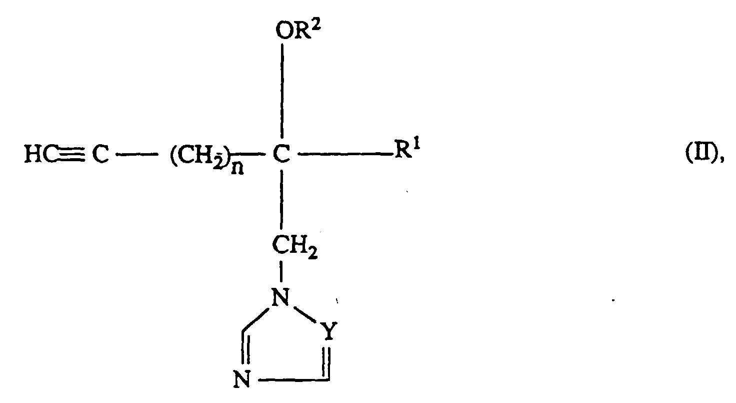 in welcher
R¹, R², Y und n die oben angegebenen Bedeutungen haben,
mit Halogen oder Halogen liefernden Verbindungen in Gegenwart eines Verdünnungsmittels umsetzt,
und gegebenenfalls anschließend an die so erhaltenen Verbindungen der Formel (I) eine Säure oder ein Metallsalz addiert

Schließlich wurde gefunden, daß die neuen Halogenalkyl-azolyl-Derivate der Formel (I) sowie deren Säureadditions-Salze und Metallsalz-Komplexe starke mikrobizide Eigenschaften besitzen und sowohl im Pflanzenschutz als auch im Materialschutz eingesetzt werden können.

Überraschenderweise besitzen die erfindungsgemäßen Stoffe sowohl im Pflanzenschutz als auch im Materialschutz eine bessere mikrobizide Wirksamkeit als die konstitutionell ähnlichsten vorbekannten Verbindungen gleicher Wirkungsrichtung.
Bevorzugt steht
- R¹: für Methyl, Ethyl, n-Propyl, Isopropyl, n-Butyl, tert.-Butyl, tert.-Pentyl, 1-Ethyl-1-methyl-propyl, 1,1-Dimethyl-propyl, oder 1,1,2-Trimethylpropyl, wobei jeder dieser zuvor genannten Reste einfach bis dreifach, gleichartig oder verschieden substituiert sein kann durch Fluor, Chlor und/oder Brom, oder für 1-Methyl-cyclohexyl, Cyclohexyl, 1-Fluor-cyclopropyl, 1-Chlor-cyclopropyl, 1-Methylcyclopropyl, Cyclopropyl, 1-Metylcyclopentyl, Cyclopentyl oder 1-Ethyl-cyclopentyl.
- R²: steht bevorzugt für Wasserstoff, Methyl, Ethyl, n-Propyl, Isopropyl, n-Butyl, Allyl, Formyl, Acetyl, Benzyl oder Phenethyl.
- X¹: steht bevorzugt für Fluor, Chlor oder Brom.
- X²: steht bevorzugt für Fluor, Chlor oder Brom.

Der Index n steht für 0 oder 1.
- Y: steht für Stickstoff oder eine CH-Gruppe.

Bevorzugte erfindungsgemäße Stoffe sind auch Additionsprodukte aus Säuren und Halogenalkyl-azolyl-Derivalen der Formel (I), in denen R¹, R², X¹, X², Y und n die Bedeutungen haben, die bereits als bevorzugt für diese Reste und den Index n genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bemsteinsäure, Fumarsäure, Weinsäure, Zitronensäure, Salizylsäure, Sorbinsäure und Milchsäure sowie Sulfonsäuren, wie z.B. p-Toluolsulfonsäure, 1,5-Naphthalindisulfonsäure, Camphersulfonsäure oder Saccharin.

Außerdem bevorzugte erfindungsgemäße Verbindungen sind Additionsprodukte aus Salzen von Metallen der II. bis IV. Haupt- und der I. und II. sowie IV. bis VIII. Nebengruppe des Periodensystems der Elemente und Halogenalkyl-azolyl-Derivaten der Formel (I), in denen R¹, R², X¹, X², Y und n die Bedeutungen haben, die bereits als bevorzugt für diese Reste und den Index n genannt wurden. Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Als Beispiele für erfindungsgemäße Stoffe seien die in der folgenden Tabelle aufgeführten Halogenalkyl-azolyl-Derivate genannt.

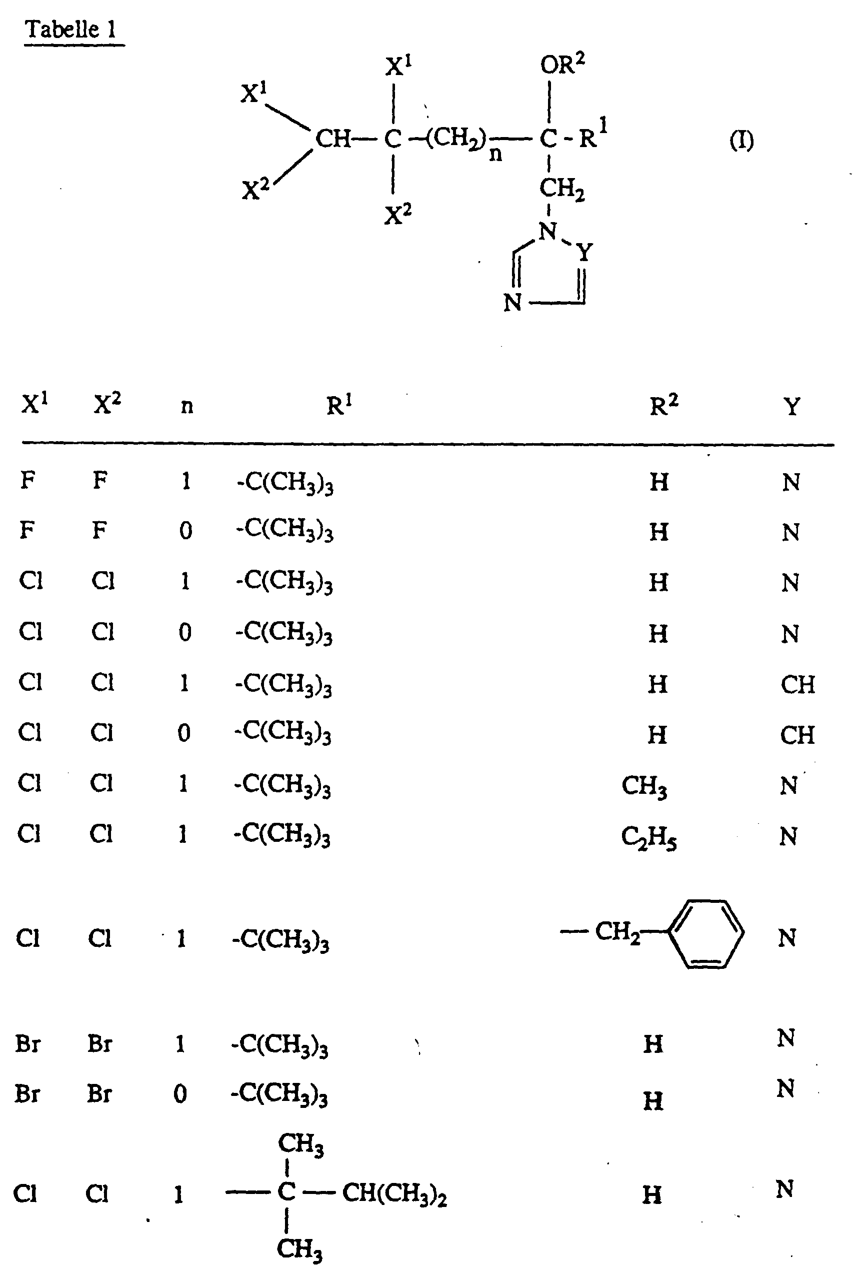

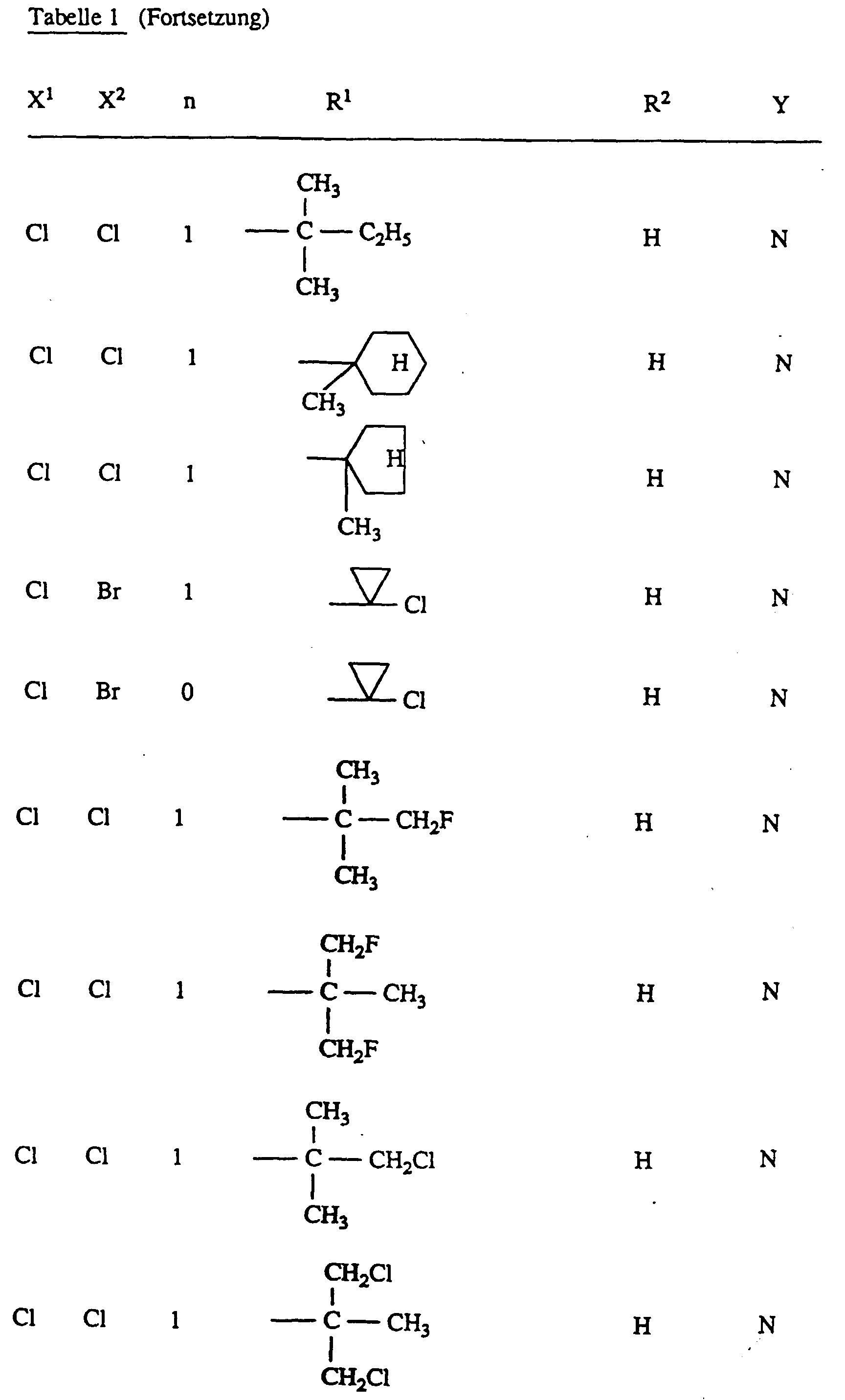

Verwendet man 3-(1-Chlor-cyclopropyl)-3-hydroxy-4-(1,2,4-triazol-1-yl)-but-1-in als Ausgangsstoff und Chlorgas als Reaktionskomponente, so kann der Verlauf des erfindungsgemäßen Verfahrens durch das folgende Formelschema veranschaulicht werden:

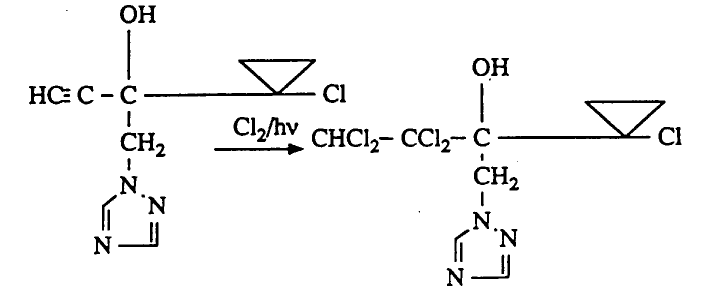

Die bei der Durchführung des erfindungsgemäßen Verfahrens als Ausgangsstoffe benötigten Alkine der Formel (II) sind teilweise bekannt (vgl. EP-A 0 096 786 und EP- A 0 353 558). Sie lassen sich herstellen, indem man
a) Azolyl-methyl-ketone der Formel 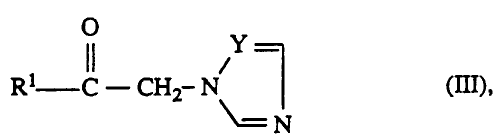 in welcher
R¹ und Y die oben angegebenen Bedeutungen haben,
entweder
α) mit Acetylen-Salzen der Formel

HC≡CMe (IV),

in welcher
Me für ein Äquivalent eines Metallkations steht,
in Gegenwart eines Verdünnungsmittels umsetzt,
oder
β) mit Propargylhalogeniden der Formel

HC≡C-CH₂-Hal (V),

in welcher
Hal für Chlor oder Brom steht,
in Gegenwart von aktiviertem Aluminium und in Gegenwart eines Verdünnungsmittels umsetzt,
und gegebenenfalls die dabei entstehenden Alkine der Formel 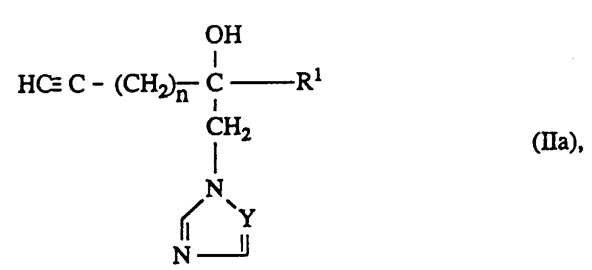 in welcher
R¹, Y und n die oben angegebenen Bedeutungen haben,
mit starken Basen in Gegenwart eines Verdünnungsmittels umsetzt und die dabei entstehenden Alkoholate der Formel 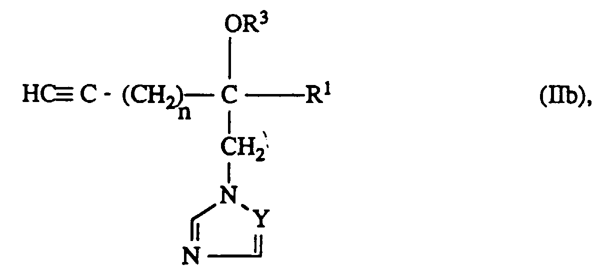 in welcher
R¹, Y und n die oben angegebenen Bedeutungen haben und
R³ für einen kationischen Rest einer Base steht,
mit Halogen-Verbindungen der Formel

R⁴-Hal' (VI),

in welcher
R⁴ für Alkyl mit 1 bis 6 Kohlenstoffatomen, Alkenyl mit 3 bis 6 Kohlenstoffatomen, Acyl mit 1 bis 4 Kohlenstoffatomen oder Phenylalkyl mit 1 bis 4 Kohlenstoffatomen im Alkylteil steht,
und
Hal' für Chlor, Brom oder Iod steht,
in Gegenwart eines Verdünnungsmittels umsetzt,
oder
b) Chlormethylketone der Formel 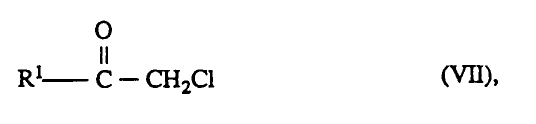 in welcher
R¹ die oben angegebene Bedeutung hat,
entweder
α) mit Acetylenen der Formel

HC≡CR⁵ (VIII),

in welcher
R⁵ für Wasserstoff oder ein Äquivalent eines Metallkations steht,
gegebenenfalls in Gegenwart einer Base und in Gegenwart eines Verdünnungsmittels umsetzt,
oder
β) mit Propargylhalogeniden der Formel

HC≡C-CH₂-Hal (V),

in welcher
Hal die oben angegebene Bedeutung hat,
unter den Bedingungen des Verfahrens (a), Variante β, umsetzt,
und dann die dabei entstehenden Hydroxyalkine der Formel 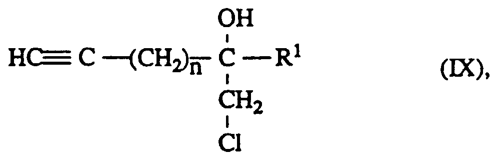 in welcher
R¹ und n die oben angegebenen Bedeutungen haben,
mit Azolen der Formel 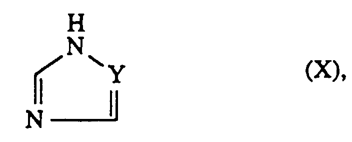 in welcher
- Y: die oben angegebene Bedeutung hat,
in Gegenwart eines Säurebindemittels und in Gegenwart eines Verdünnungsmittels umsetzt und gegebenenfalls die dabei entstehenden Alkine der Formel 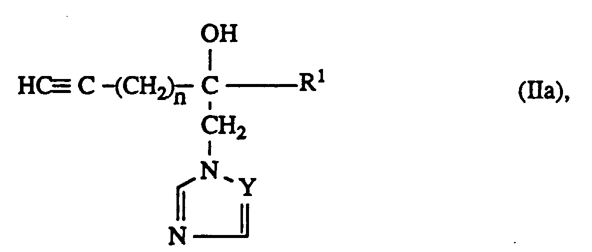 in welcher R¹, Y und n die oben angegebenen Bedeutungen haben,
gemäß Verfahren (a) weiter umsetzt.

Die bei der Durchführung des Verfahrens (a) als Ausgangsstoffe benötigten Azolyl-methyl-ketone der Formel (III) sind bekannt oder lassen sich nach prinzipiell bekannten Verfahren in einfacher Weise herstellen (vgl. DE-A 2 431 407 und EP-A 0353558).

Die bei der Durchführung des Verfahrens (a), Variante α, als Reaktionskomponenten benötigten Acetylen-Salze sind durch die Formel (IV) allgemein definiert. In dieser Formel steht Me vorzugsweise für ein Lithiumkation oder für ein Äquivalent eines Cer (III)-Kations.

Die Acetylen-Salze der Formel (IV) sind bekannt (vgl. Houben-Weyl, "Methoden der Organischen Chemie", Bd. V/2a, Seiten 509 ff., Georg Thieme Verlag, Stuttgart 1977 und Tetrahedron Letters 25, (1984) 4233).

Als Verdünnungsmittel können bei der Durchführung der ersten Stufe des Verfahrens (a), Variante α, alle für derartige Umsetzungen üblichen inerten organischen Solventien verwendet werden. Vorzugsweise in Frage kommen Ether, wie Tetrahydrofuran oder Diethylether, und außerdem Kohlenwasserstoffe, wie n-Hexan.

Die Reaktionstemperaturen können bei der Durchführung der ersten Stufe des Verfahrens (a), Variante α, innerhalb eines größeren Bereiches variiert werden. Im allgemeinen arbeitet man bei Temperaturen zwischen -78°C und +30°C, vorzugsweise bei Temperaturen zwischen -70°C und +20°C.

Bei der Durchführung des Verfahrens (a) arbeitet man ebenso wie bei der Durchführung des erfindungsgemäßen Verfahrens und des Verfahrens (b) im allgemeinen unter Normaldruck.

Man geht bei der Durchführung der ersten Stufe des Verfahrens (a), Variante α, im allgemeinen so vor, daß man zunächst die Acetylen-Salze herstellt und diese dann ohne vorherige Isolierung mit einer äquivalenten Menge, bzw. einem Über- oder Unterschuß an Azolyl-methyl-keton der Formel (III) umsetzt Die Aufarbeitung erfolgt nach üblichen Methoden. Im allgemeinen geht man so vor, daß man das Reaktionsgemisch zunächst mit einer wäßrigen Salz-Lösung, z.B. Ammoniumchlorid-Lösung, versetzt, dann mehrfach mit einem in Wasser wenig löslichen organischen Solvens ausschüttelt und die vereinigten organischen Phasen nach dem Trocknen unter vermindertem Druck einengt

Die bei der Durchführung des Verfahren (a), Variante β, als Reaktionskomponenten benötigten Propargylhalogenide der Formel (V) sind bekannt.

Als Verdünnungsmittel können bei der Durchführung der ersten Stufe des Verfahrens (a), Variante β, alle für derartige Umsetzungen üblichen inerten organischen Solventien verwendet werden. Vorzugsweise in Frage kommen Ether, wie Tetrahydrofuran oder Diethylether.

Bei der Durchführung der ersten Stufe des Verfahrens (a), Variante β, arbeitet man in Gegenwart von aktiviertem Aluminium. Dieses wird hergestellt, indem man zu Aluminium-Schuppen katalytische Mengen an Quecksilber-(II)-chlorid und Iod zugibt

Die Reaktionstemperaturen können bei der Durchführung der ersten Stufe des Verfahrens (a), Variante β, innerhalb eines größeren Bereiches variiert werden. Im allgemeinen arbeitet man bei Temperaturen zwischen -80°C und +100°C, vorzugsweise bei Temperaturen zwischen -70°C und +60°C.

Man geht bei der Durchführung der ersten Stufe des Verfahrens (a), Variante β, im allgemeinen so vor, daß man auf 1 Mol an Azolyl-methyl-keton der Formel (III) 1 bis 2 Mol Propargyl-halogenid der Formel (V) und 1 bis 1,5 Mol Aluminium und katalytische Mengen an Quecksilber-(II)-chlorid und Iod einsetzt. Die Isolierung der entstehenden Produkte erfolgt nach üblichen Methoden.

Bei der Durchführung der zweiten Stufe des Verfahrens (a) erfolgt die Überführung der Alkine der Formel (IIa) in die entsprechenden Alkoholate, indem man mit geeigneten starken Basen, wie Alkalimetall-amiden oder -hydriden, quarternären Ammonium-hydroxiden oder Phosphonium-hydroxiden in einem inerten Verdünnungsmittel, wie z.B. Dioxan, bei Raumtemperatur umsetzt. Demgemäß steht R³ in den Verbindungen der Formel (IIb) vorzugsweise für ein Alkalimetallkation, wie ein Natrium- oder Kaliumkation, oder für ein quarternäres Ammonium- oder Phosphoniumkation.

Die bei der Durchführung der dritten Stufe des Verfahrens (a) als Reaktionskomponenten benötigten Halogen-Verbindungen sind durch die Formel (VI) allgemein definiert In dieser Formel steht R⁴ vorzugsweise für die Bedeutungen, die bereits im Zusammenhang mit der Beschreibung der erfindungsgemäßen Stoffe der Formel (I) für den Substituenten R² genannt wurden mit Ausnahme der Bedeutung von Wasserstoff. Hal' steht für Chlor, Brom oder Iod. Die Halogenverbindungen der Formel (VI) sind bekannt oder lassen sich nach im Prinzip bekannten Methoden herstellen.

Als Verdünnungsmittel kommen bei der Durchführung der zweiten und drinen Stufe des Verfahrens (a) inerte organische Lösungsmittel in Frage. Vorzugsweise verwendbar sind Ether, wie Diethylether oder Dioxan; aromatische Kohlenwasserstoffe, wie Benzol; in einzelnen Fällen auch chlorierte Kohlenwasserstoffe, wie Chloroform, Methylenchlorid oder Tetrachlorkohlenstoff; sowie Hexamethylphosphorsäuretriamid. Die Reaktionstemperaturen können bei der Durchführung der zweiten und dritten Stufe des Verfahrens (a) in einem größeren Bereich variiert werden. Im allgemeinen arbeitet man bei Temperaturen zwischen 0°C und 120°C, vorzugsweise zwischen 20°C und 100°C.

Bei der Durchführung der zweiten Stufe des Verfahrens (a) setzt man zunächst Alkine der Formel (IIa) mit starken Basen zu den entsprechenden Alkoholaten der Formel (IIb) um. In der folgenden dritten Stufe setzt man auf 1 Mol eines Alkoholates der Formel (IIb) vorzugsweise 1 bis 2 Mol Halogenverbindung der Formel (VI) ein. Zur Isolierung der Endprodukte wird das Reaktionsgemisch vom Lösungsmittel befreit und der Rückstand mit Wasser und einem organischen Lösungsmittel versetzt. Die organische Phase wird abgetrennt, in üblicher Weise aufgearbeitet und gereinigt.

In einer bevorzugten Ausführungsform wird in der zweiten und dritten Stufe des Verfahrens (a) zweckmäßigerweise so verfahren, daß man von einer Hydroxy-Verbindung der Formel (IIa) ausgeht, letztere in einem geeigneten organischen Lösungsmittel mittels Alkalimetall-hydrid oder Alkalimetall-amid in das Alkalimetallalkoholat überführt und letzteres ohne Isolierung sofort mit einer Halogenverbindung der Formel (VI) umsetzt, wobei unter Austritt von Alkalimetallhalogenid die Verbindungen der Formel (II) in einem Arbeitsgang erhalten werden.

Nach einer weiteren bevorzugten Ausführungsform werden zweckmäßigerweise die Herstellung der Alkoholate sowie die Umsetzung mit einer Halogenverbindung der Formel (VI) in einem Zweiphasensystem, wie beispielsweise wäßrige Natron- oder Kalilauge/Toluol oder Methylenchlorid, unter Zusatz von 0,01 bis 1 Mol eines Phasen-Transfer-Katalysators, wie beispielsweise Ammonium- oder Phosphoniumverbindungen, durchgeführt, wobei in der organischen Phase oder an der Grenzfläche die Alkoholate mit den in der organischen Phase befindlichen Halogeniden umgesetzt werden.

Die bei der Durchführung des Verfahrens (b) als Ausgangsstoffe benötigten Chlormethylketone sind durch die Formel (VII) allgemein definiert In dieser Formel hat R¹ vorzugsweise diejenigen Bedeutungen, die bereits im Zusammenhang mit der Beschreibung der erfindungsgemäßen Stoffe der Formel (I) vorzugsweise für diesen Rest genannt wurden.

Die Chlormethylketone der Formel (VII) sind bekannt oder lassen sich nach prinzipiell bekannten Methoden herstellen (vgl. DE-A 3 049 461).

Die bei dem Verfahren (b), Variante α, als Reaktionskomponenten benötigten Acetylene sind durch die Formel (VIII) allgemein definiert. In dieser Formel steht R⁵ vorzugsweise für Wasserstoff, ein Lithium-kation oder ein Äquivalent eines Magnesium- oder Cer (III)-Kations.

Die Acetylene der Formel (VIII) sind bekannt.

Als Basen kommt bei der Durchführung der ersten Stufe des Verfahrens (b), Variante α, alle für derartige Umsetzungen üblichen starken Basen in Betracht. Vorzugsweise verwendbar sind Alkalimetallhydroxide, wie Kaliumhydroxid.

Als Verdünnungsmittel können bei der Durchführung der ersten Stufe des Verfahrens (b), Variante α, alle für derartige Umsetzungen üblichen inerten organischen Solventien verwendet werden. Vorzugsweise in Frage kommen, Ether, wie Tetrahydrofuran oder Diethylether.

Die Reaktionstemperaturen können bei der Durchführung der ersten Stufe des Verfahrens (b), Variante α, innerhalb eines größeren Bereichs variiert werden. Im allgemeinen arbeitet man bei Temperaturen zwischen -78°C und +50°C, vorzugsweise zwischen -78°C und +40°C.

Bei der Durchführung der ersten Stufe des Verfahrens (b), Variante α, geht man im allgemeinen so vor, daß man Chlormethylketone der Formel (VII) und Acetylene der Formel (VIII) in angenähert äquivalenten Mengen umsetzt. Es ist jedoch auch möglich, die eine oder andere Komponente in einem Überschuß zu verwenden. Die Aufarbeitung erfolgt nach üblichen Methoden.

Die Durchführung der ersten Stufe des Verfahrens (b), Variante β, erfolgt unter den Bedingungen, die auch bei der Durchführung der ersten Stufe des Verfahrens (a), Variante β, angewandt werden.

Die Hydroxyalkine der Formel (IX) können direkt weiter umgesetzt werden mit Azolen der Formel (X). Sie können aber auch zunächst in Oxirane überführt werden und dann mit Azolen der Formel (X) umgesetzt werden.

Bei der Durchführung der zweiten Stufe des Verfahrens (b) kommen als Säurebindemittel alle üblichen Säureakzeptoren in Frage. Vorzugsweise verwendbar sind Alkalimetallcarbonate und Hydrogencarbonate, wie Natriumcarbonat, Kaliumcarbonat oder Natriumhyrogencarbonat, ferner tertiäre aliphatische oder aromatische Amine, wie Triethylamin, N,N-Dimethyl-cyclohexyl-amin, N,N-Dimethyl-benzylamin und Pyridin, und außerdem cyclische Amine, wie 1,5-Diaza-bicyclo-[4.3.0]non-5-en (DBN), 1,8-Diaza-bicyclo[5.4.0]undec-7-en (DBU) und 1,4-Diaza-bicyclo[2.2.2]octan (DABCO).

Als Verdünnungsmittel kommen bei der Durchführung der zweiten Stufe des Verfahrens (b) alle inerten organischen Solventien in Betracht. Vorzugsweise verwendbar sind aliphatische und aromatische, gegebenenfalls halogenierte Kohlenwasserstoffe wie Pentan, Hexan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, sowie tert.-Butylmethylether, Glykoldimethylether und Diglykoldimethylether, Tetrahydrofuran und Dioxan, Ketone wie Aceton, Methyl-ethyl-, Methyl-isopropyl- und Methyl-isobutyl-keton. Ester wie Essigsäuremethylester und -ethylester, Nitrile wie z.B. Acetonitril und Propionitril, und Pyridin.

Die Reaktionstemperaturen können auch bei der Durchführung der zweiten Stufe des Verfahrens (b) innerhalb eines größeren Bereiches variiert werden. Im allgemeinen arbeitet man bei Temperaturen zwischen 0°C und 200°C, vorzugsweise zwischen 20°C und 150°C.

Bei der Durchführung der zweiten Stufe des Verfahrens (b) geht man im allgemeinen so vor, daß man auf 1 Mol an Hydroxyalkin der Formel (IX) eine äquivalente Menge oder auch einen Überschuß an Azol der Formel (X) sowie 2 bis 3 Mol an Säurebindemittel einsetzt Die Aufarbeitung erfolgt nach üblichen Methoden. Die gegebenenfalls gewünschte weitere Umsetzung der Alkine der Formel (IIa) erfolgt bei dem Verfahren (b) in gleicher Weise wie bei dem Verfahren (a).

Als Halogene kommen bei der Durchführung des erfindungsgemäßen Verfahrens (a) vorzugsweise Fluor, Chlor und Brom als Reaktionskomponenten in Betracht, ferner gemischte Halogene wie Chlor(I)-fluorid, Brom(I)-fluorid oder Brom(I)-chlorid, (s. Methodicum Chimicum, F. Korte, Bd. 7, S. 842 (1976)).

Als Halogen liefernde Verbindungen können beispielsweise Sulfurylchlorid, N-Bromsuccinimid mit Salzsäure, N-Chlorsuccinimid mit Bromwasserstoffsäure oder N-Chlorsuccinimid mit Fluorwasserstoff/Pyridin (s. Synthesis 1973, 780) verwendet werden.

Die Addition der Halogene an die Alkine der Formel (II) kann durch Einwirkung von Licht, durch Wärme, durch radikalbildende Substanzen, wie organische Peroxide, durch oberflächenaktive Stoffe, wie Aktivkohle, oder Metallsalz, wie Kupfer(II)-chlorid oder Eisen(III)-chlorid, begünstigt werden.

Als Verdünnungsmittel können bei der Durchführung des erfindungsgemäßen Verfahrens alle für derartige Umsetzungen üblichen inerten Solventien eingesetzt werden. Vorzugsweise verwendbar sind halogenierte aliphatische Kohlenwasserstoffe, wie Methylenchlorid, Chloroform und Tetrachlorkohlenstoff, oder auch Phosphoroxychlorid.

Die Temperaturen können bei der Durchführung des erfindungsgemäßen Verfahrens innerhalb eines bestimmten Bereiches variiert werden. Im allgemeinen arbeitet man bei Temperaturen zwischen -10°C und +120°C, vorzugsweise zwischen -5°C und +80°C.

Bei der Durchführung des erfindungsgemäßen Verfahrens setzt man auf 1 Mol an Alkin der Formel (II) im allgemeinen eine äquivalente Menge oder einen Überschuß an Halogen bzw. Halogen liefernder Verbindung ein. Wenn es beabsichtigt ist, Halogenalkyl-azolyl-Derivate der Formel (I) herzustellen, in denen X¹ und X² verschieden sind, so geht man zweckmäßigerweise so vor, daß man zunächst 1 Mol eines bestimmten Halogens an das Alkin der Formel (II) addiert und danach in einem zweiten Schritt 1 Mol eines anderen Halogens addiert. Die Aufarbeitung erfolgt nach üblichen Methoden. Im allgemeinen geht man so vor, daß man mit einem in Wasser wenig löslichen organischen Solvens verdünnt, mit Wasser wäscht und die organische Phase nach dem Trocknen einengt. Es ist jedoch auch möglich, das Reaktionsgemisch nach beendeter Umsetzung direkt durch Abziehen der flüchtigen Komponenten unter vermindertem Druck einzuengen. Die entstehenden Produkte können gegebenenfalls nach üblichen Methoden weiter gereinigt werden.

Die nach dem erfindungsgemäßen Verfahren erhältlichen Halogenalkyl-azolyl-Derivate der Formel (I) können in Säureadditions-Salze oder Metallsalz-Komplexe überführt werden.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise diejenigen Salze von Metallen in Frage, die bereits im Zusammenhang mit der Beschreibung der erfindungsgemäßen Metallsalz-Komplexe als bevorzugte Metallsalze genannt wurden.

Die Metallsalz-Komplexe der Verbindungen der Formel (I) können in einfacher Weise nach üblichen Verfahren erhalten werden, so z.B. durch Lösen des Metallsalzes in Alkohol, z.B. Ethanol und Hinzufügen zu Verbindungen der Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können zur Bekämpfung unerwünschter Mikroorganismen, wie Fungi und Bakterien, im Pflanzenschutz und im Materialschutz eingesetzt werden.

Fungizide werden im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;
Phytophthora-Arten, wie Phytophthora infestans;
Pseudoperonospora-Arten, wie Pseudoperonospora humuli oder Pseudoperonospora cubensis;
Plasmopara-Arten, wie Plasmopara viticola;
Peronospora-Arten, wie Peronospora pisi oder P. brassicae;
Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Venturia inaequalis;
Pyrenophora-Arten, wie Pyrenophora teres oder P. graminea;
(Konidienform: Drechslera, Syn: Helminthosporium);
Cochliobolus-Arten, wie Cochliobolus sativus;
(Kondienform: Drechslera, Syn: Helminthosporium);
Uromyces-Arten, wie Uromyces appendiculatus;
Puccinia-Arten, wie Puccinia recondita;
Tilletia-Arten, wie Tilletia caries;
Ustilago-Arten, wie Ustilago nuda oder Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;
Altemaria-Arten, wie Alternaria brassicae;
Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Die erfindungsgemäßen Wirkstoffe eignen sich insbesondere zur Bekämpfung von Getreide- und Reiskrankheiten, wie Pseudocercosporella, Erysiphe, Fusarium, Pyrenophora, Cochliobolus, Septoria, Pyricularia und Pellicularia, sowie zur Bekämpfung von Gurkenmehltau und Apfelschorf und außerdem zur Bekämpfung von Botrytis im Obst-, Wein- und Gemüsebau. Sie besitzen außerdem eine gute und breite in - vitro - Wirkung und eignen sich zur Bekämpfung echter Mehltaupilze, wie Rhizoctonia solani.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nicht-lebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa
Staphylococcus, wie Staphylococcus aureus.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmittel oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im Pflanzenschutz im allgemeinen zwischen 0,1 und 95 Gewichtsprozent Wirkstoff, vorzugsweise zwischen 0,5 und 90 %.

Die erfindungsgemäßen Wirkstoffe können bei Verwendung im Pflanzenschutz in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe können bei Verwendung im Pflanzenschutz als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angwendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Die zum Schutz technischer Materialien verwendeten mikrobiziden Mittel enthalten die Wirkstoffe im allgemeinen in einer Menge von 1 bis 95 Gew.-%, bevorzugt von 10 bis 75 Gew.-%.

Beim Einsatz im Materialschutz richten sich die Anwendungskonzentrationen an erfindungsgemäßen Wirkstoffen nach der Art und dem Vorkommen der zu bekämpfenden Mikroorganismen sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gew.-%, vorzugsweise von 0,05 bis 1,0 Gew.-%, bezogen auf das zu schützende Material.

Auch beim Einsatz im Materialschutz können die erfindungsgemäßen Wirkstoffe in Mischung mit anderen bekannten Wirkstoffen angewendet werden.

Beispielsweise seien die folgenden Wirkstoffe genannt:
Benzylalkoholmono(poly)hemiformal und andere Formaldehyd abspaltende Verbindungen, Benzimidazolyl-methylcarbamate, Tetramethylthiuramdisulfid, Zinksalze von Dialkyldithiocarbamaten, 2,4,5,6-Tetrachlorisophthalonitril, Thiazolylbenzimidazol, Mercaptobenzthiazol, 2-Thiocyanatomethylthiobenzthiazol, Methylenbisthiocyanat, Phenolderivate wie 2-Phenylphenol, (2,2'-Dihydroxy-5,5'-dichlor)-diphenylmethan und 3-Methyl-4-chlor-phenol, Organo-Zinnverbindungen, Trihalogenmethylthio-Verbindungen wie Folpet, Fluorfolpet, Dichlofluanid.

Die Herstellung und die Verwendung der erfindungsgemäßen Wirkstoffe werden durch die folgenden Beispiele veranschaulicht

### Herstellungsbeispiele

### Beispiel 1

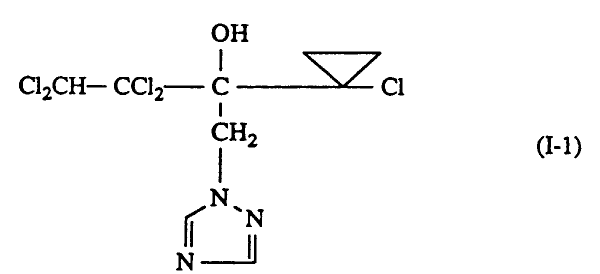

1,06 g (5 mMol) 3-(1-Chlor-cyclopropyl)-3-hydroxy-4-(1,2,4-triazol-1-yl)-but-1-in werden in 40 ml Methylenchlorid bei 20°C unter Belichtung mit einer 300 Watt UV-Lampe durch Einleiten von Chlorgas chloriert. Die Reaktionsmischung wird zur Aufarbeitung mit Methylenchlorid verdünnt und mit wäßriger Natriumcarbonat-Lösung ausgeschüttelt. Die organische Phase wird nach dem Trocknen über Natriumsulfat unter vermindertem Druck eingeengt Nach chromatographischer Reinigung des Rohproduktes erhält man 3-(1-Chlor-cyclopropyl)-3-hydroxy-4-(1,2,4-tfiazol-1-yl)-1,1,2,2-tetrachlorbutan in Form einer Festsubstanz vom Schmelzpunkt 130°C.
¹H-NMR (200 MHz; CDCl₃):
- δ=: 0,2 (m; 1H), 0,5 (m; 1H), 1,1 (m; 1H), 1,5 (m,lH) 5,25 (AB,2H), 5,35 (OH), 6,5 (s,1H), 8,05 (s,1H), 8,35 (s,1H).

### Herstellung der Ausgangssubstanz:

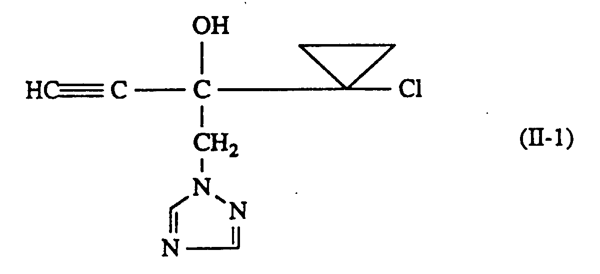

Ein Gemisch aus 18,0 g (0,1 Mol) 4-Chlor-3-(1-chlor-cyclopropyl)-3-hydroxy1-butin, 13,9 g (0,201 Mol) 1,2,4-Triazol und 55,5 g (0,402 Mol) Kaliumcarbonat in 250 ml absolutem Aceton wird 2 Stunden unter Rückfluß erhitzt. Anschließend wird durch Abziehen des Verdünnungsmittels unter vermindertem Druck eingeengt und dann mit Wasser versetzt. Das entstehende Gemisch wird mit Methylenchlorid extrahiert. Die vereinigten organischen Phasen werden getrocknet und unter vermindertem Druck eingeengt. Der verbleibende Rückstand wird mit einem Gemisch aus Cyclohexan/Ethylacetat = 1:1 als Laufmittel an Kieselgel chromatographiert. Nach dem Einengen des Eluates erhält man 9,7 g (46 % der Theorie)an 3-(1-Chlor-3-(1-Chlor-1-cyclopropyl)-3-hydroxy-4-(1,2,4-triazol-1-yl)-but-1-in Form einer Festsubstanz vom Schmelzpunkt 70-72°C.

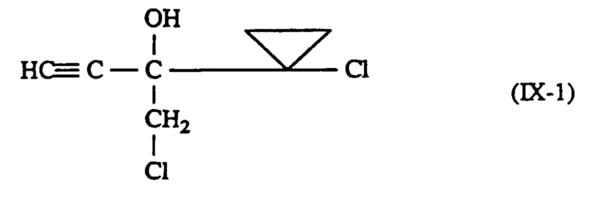

Zu einer Lösung von 5,2 g (0,2 Mol) Acetylen in 200 ml absolutem Tetrahydrofuran werden bei 70°C 80 ml (0,2 Mol) 23 %-ige Butyllithiumlösung in Hexan zugetropft

Nach 10 Minuten wird eine Lösung von 20 g (0,13 Mol) 2-Chlor-1-(1-chlorcyclopropyl-1-)-ethanon in 100 ml absolutem Tetrahydrofuran zugefügt. Es wird 2 Stunden bei -70°C nachgerührt. Danach wird die Reaktion durch Zutropfen des Gemisches aus 20 ml Methanol und 20 ml Essigsäure gestoppt.Das Lösungsmittel wird im Vakuum abgezogen und der Rest nach Lösen in Dichlormethan mit gesättigter, wäßriger Ammoniumchloridlösung mehrfach ausgeschüttelt. Nach dem Trocknen und Abdestillieren der organischen Phase erhält man 24,4 g Rohprodukt mit einem Gehalt von 83,1 % an 4-Chlor-3-(1-chlor-cyclopropyl)-3-hydroxy-1-butin.
¹H-NMR (200 MHz; CDCl₃)
- δ=: 1,1 (m, 2H); 1,4 (m, 2H); 2,55 (s, 1H); 3,3 (OH); 3,95 (AB-System, 2H).

### Beispiel 2

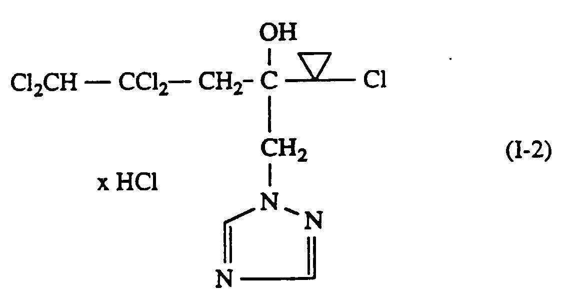

In eine Lösung von 30 g (0,133 Mol) 4-(1-Chlor-cyclopropyl)-4-hydroxy-5-(1,2,4-triazol-1-yl)-pent-1-in in 500 ml Methylenchlorid werden bei Temperaturen zwischen 5 und 10°C unter Belichtung mit einer 300 Watt UV-Lampe innerhalb von 4,5 Stunden 0,5 Mol Chlorgas eingeleitet. Danach läßt man das Reaktionsgemisch 70 Stunden bei Raumtemperatur stehen und arbeitet dann auf, indem man den ausgefallenen Niederschlag absaugt und unter vermindertem Druck trocknet. Man erhält auf diese Weise 40 g (74 % der Theorie) an 4-(1-Chlor-cyclopropyl)-4-hydroxy-5-(1,2,4-triazol-1-yl)-1,1,2,2-tetrachlorpentan-Hydrochlorid in Form einer Festsubstanz mit dem Schmelzpunkt 184 bis 185°C.
¹H-NMR (200 MHz-CDCl₃):
- δ=: 0,4 (m; 2H), 0,85 (m; 1H), 1,1 (m; 1H), 3,2 (AB-System; 2H), 4,75 (OH), 4,95 (AB-System; 2H), 6,65 (s; 1H), 8,05 (s; 1H), 8,45 (s; 1H).

### Herstellung der Ausgangssubstanz:

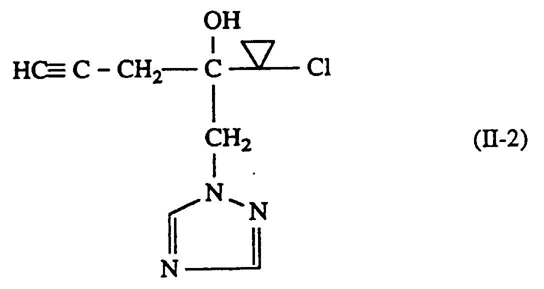

5,5 g (0,204 Mol) Aluminiumschuppen werden mit einer Spatelspitze Quecksilber-(II)chlorid und Iod versetzt und eine Stunde lang bei 40°C in 25 ml Tetrahydrofuran gerührt. Anschließend wird auf Rückflußtemperatur erwärmt, eine Lösung aus 35,0 g (0,294 Mol) Propargylbromid in 38 ml Tetrahydrofuran zugetropft und 30 Minuten nachgerührt. Es wird auf -60°C gekühlt und eine Lösung von 25 g (0,135 Mol) 1-Chlor-1-[(1,2,4-triazol-1-yl)-acetyl]-cyclopropan in 75 ml Tetrahydrofuran zugetropft, eine Stunde bei 0°C und zwei Stunden bei Raumtemperatur nachgerührt. Danach wird mit 75 ml gesättigter wäßriger Ammoniumchloridlösung versetzt, filtriert und eingeengt. Der Rückstand wird mit Ethylacetat extrahiert, die organische Phase mit Wasser gewaschen, über Natriumsulfat getrocknet und eingeengt. Dabei fallen 30,5 g (100 %der Theorie) Rohprodukt an.

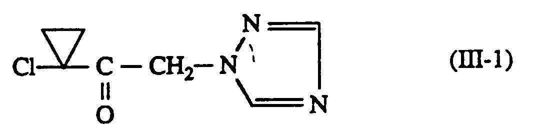

Zu einer unter Rückfluß kochenden Suspension von 83 g (0,6 Mol) Kaliumcarbonat und 58 g (0,84 Mol) Triazol in 330 ml Acetonitril werden 100 g (0,66 Mol) 1-Chlor-cyclopropyl-chlormethyl-keton in 80 ml Acetonitril zugetropft. Es wird acht Stunden unter Rückfluß gekocht, dann abgesaugt und eingeengt. Der Rückstand wird in Ethylacetat/Toluol aufgenommen, mit Wasser gewaschen, über Natriumsulfat getrocknet und eingeengt. Die anschließende säulenchromatographische Reinigung mit dem Laufmittel Dichlormethan lieferte 62 g (51 % der Theorie) 1-Chlor-1-[(1,2,4-triazol-1-yl)-acetyl]-cyclopropan.

### Beispiel 3

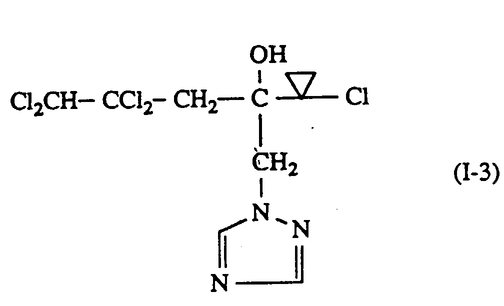

Durch Behandlung der Verbindung der Formel (I-2) mit wäßriger Natriumcarbonatlösung erhält man die Verbindung der Formel (I-3) in Form einer Feststubstanz vom Schmelzpunkt 131-133°C.

Nach der im Beispiel 2 angegebenen Methode werden auch die in den folgenden Beispielen aufgeführten Stoffe hergestellt.

### Beispiel 4

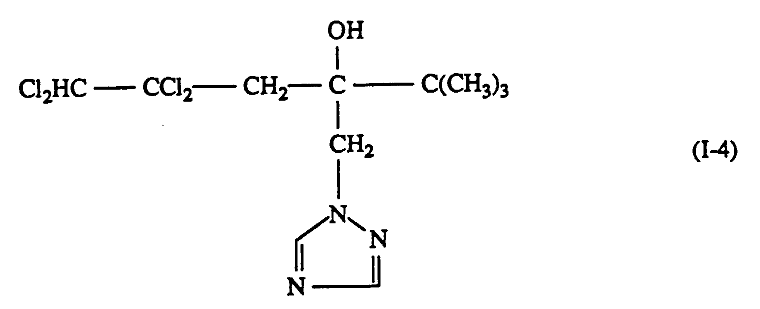

Schmelzpunkt: 137-138°C

### Beispiel 5

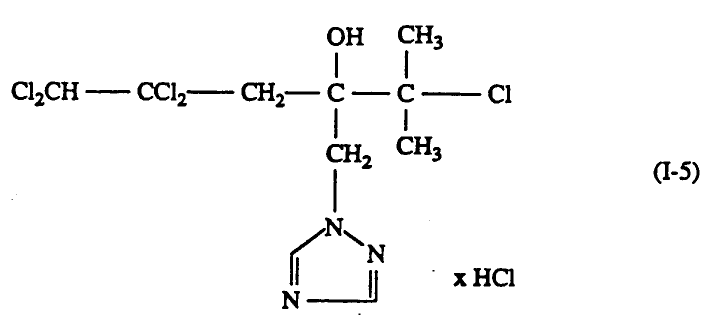

¹H-NMR (200 MHz, CDCl₃):
- δ=: 1,75 (s, 3H), 1,8 (s, 3H), 3,2 (AB, 2H), 5,2 (AB, 2H), 6,2 (s, 1H), 8,5 (s, 1H), 10,5 (s, 1H).

### Beispiel 6

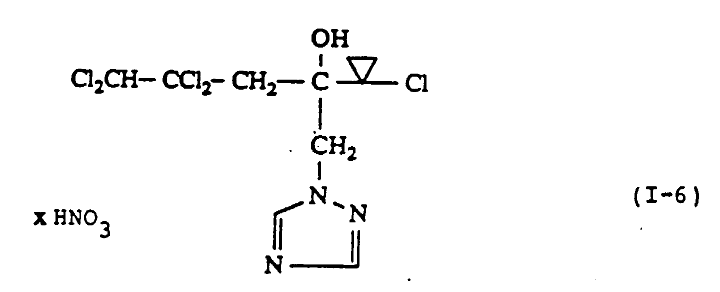

Schmelzpunkt: 152-154°C

In den folgenden Verwendungsbeispielen wurden die Verbindungen der nachstehend aufgeführten Formeln als Vergleichssubstanzen eingesetzt: 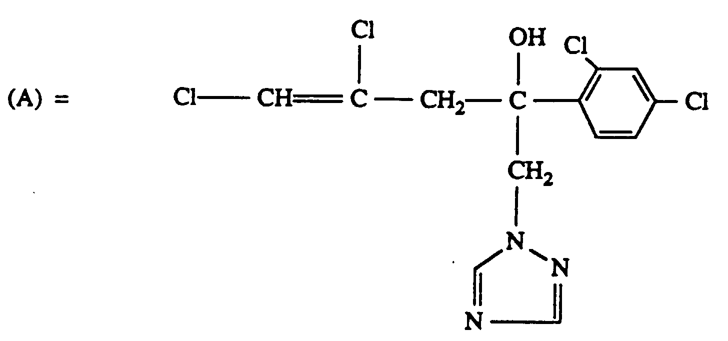 (Bekannt aus EP-A 0 097 425). 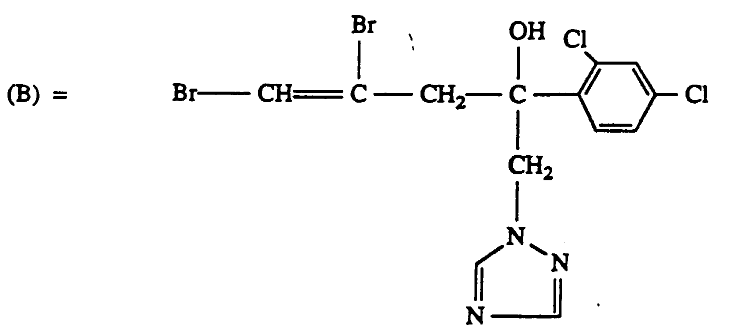 (Bekannt aus EP-A 0 097 425).

### Beispiel A

Erysiphe-Test (Gerste)/protektiv
- Lösungsmittel:: 100 Gewichtsteile Dimethylformamid
- Emulgator:: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20°C und einer relativen Luftfeuchtigkeit von ca 80 % aufgestellt, um die Entwicklung von Mehltaupusteln zu begünstigen.

7 Tage nach der Inokulation erfolgt die Auswertung.

In diesem Test zeigt der erfindungsgemäße Stoff (I-2) eine wesentlich bessere Wirksamkeit als die Vergleichssubstanzen (A) und (B).

### Beispiel B

Erysiphe-Test (Weizen)/protektiv
- Lösungsmittel:: 100 Gewichtsteile Dimethylformamid
- Emulgator:: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.tritici bestäubt.

Die Pflanzen werden in einem Gewächshaus bei einer Temperatur von ca 20°C und einer relativen Luftfeuchtigkeit von ca 80 % aufgestellt, um die Entwicklung von Mehltaupusteln zu begünstigen.

7 Tage nach der Inokulation erfolgt die Auswertung.

In diesem Test zeigt die erfindungsgemäße Verbindung (I-2) eine wesentlich bessere Wirksamkeit als die Vergleichssubstanzen (A) und (B).

### Beispiel C

Pseudocercosporella herpotrichoides-Test (Weizen)/protektiv
- Lösungsmittel:: 100 Gewichtsteile Dimethylformamid
- Emulgator:: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen an der Halmbasis mit Sporen von Pseudocercosporella herpotrichoides inokuliert.

Die Pflanzen werden in einem Gewächshaus bei einer Temperatur von ca 10°C und einer relativen Luftfeuchtigkeit von ca. 80 % aufgestellt.

21 Tage nach der Inokulation erfolgt die Auswertung.

In diesem Test zeigt der erfindungsgemäße Stoff (1-2) eine wesentlich bessere Wirksamkeit als die Vergleichssubstanz (A).

### Beispiel D

Fusarium culmorum-Test / Weizen / protektiv
- Lösungsmittel:: 100 Gewichtsteile Aceton
- Emulgator:: 4,5 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeii besprüht man 7 Tage alte Pflanzen mit der Wirkstoffzubereitung bis zur Tropfnässe. Nach 24 Stunden werden die Pflanzen mit Konidien von Fusarium culmorum, welche in einer 2,4 %igen Potato-Dextrose-Broth (Difco) suspendiert sind, besprüht. Unmittelbar vorher werden die Blätter durch Nadelstiche verletzt. Die Pflanzen verbleiben anschließend bis zur Auswertung 7 Tage in einer lichtdurchlässigen Inkubationskammer, in der die Temperatur tagsüber 27°C und nachts 21°C beträgt und in der eine Luftfeuchte von 100 % herrscht.

In diesem Test zeigt die erfindungsgemäße Verbindung (I-2) eine wesentlich bessere Wirksamkeit als die Vergleichssubstanzen (A) und (B).

### Beispiel E

Gibberella zeae (= Fusarium graminearum)-Test/Gerste/protektiv
- Lösungsmittel:: 100 Gewichtsteile Aceton
- Emulgator:: 4,5 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man ca. 7 Tage alte Pflanzen mit der Wirkstoffzubereitung bis zur Tropfnässe. Nach 24 Stunden werden die Pflanzen mit Konidien von Gibberella zeae, welche in einer 2,4 %igen Potato-Dextrose-Broth (Difco) suspendiert sind, besprüht. Unmittelbar vorher werden die Blätter durch Nadelstiche verletzt. Die Pflanzen verbleiben anschließend bis zur Auswertung 7 Tage in einer lichtdurchlässigen Inkubationskammer, in der die Temperatur tagsüber 27°C und nachts 21°C beträgt und in der eine Luftfeuchte von 100 % herrscht.

In diesem Test zeigt die erfindungsgemäße Verbindung (I-2) eine wesentlich bessere Wirksamkeit als die Vergleichssubstanzen (A) und (B).

### Beispiel F

Venturia-Test (Apfel) / protektiv
- Lösungsmittel:: 4,7 Gewichtsteile Aceton
- Emulgator:: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20°C und 100 % relativer Luftfeuchtigkeit in einer Inkubationskabine.

Die Pflanzen werden dann im Gewächshaus bei 20°C und einer relativen Luftfeuchtigkeit von ca 70 % aufgestellt.

12 Tage nach der Inokulation erfolgt die Auswertung.

In diesem Test zeigt die erfindungsgemäße Verbindung (I-2) eine wesentlich bessere Wirksamkeit als die Vergleichssubstanzen (A) und (B).

### Beispiel G

Botrytis-Test (Buschbohne)/protektiv (Sporeninfektion)
- Lösungsmittel:: 4,7 Gewichtsteile Aceton
- Emulgator:: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt Die inokulierten Pflanzen werden in einer abgedunkelten, feuchten Kammer bei 20°C aufgestellt. 3 Tage nach der Inokulation wird die Größe der Befallsflecken auf den Blättern ausgewertet.

In diesem Test zeigt die erfindungsgemäße Verbindung (I-2) eine wesentlich bessere Wirksamkeit als die Vergleichssubstanzen (A) und (B).

### Beispiel H

Botrytis-Test (Buschbohne)/protektiv (Mycelinfektion)
- Lösungsmittel:: 4,7 Gewichtsteile Aceton
- Emulgator:: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20°C aufgestellt. 3 Tage nach der Inokulation wird die Größe der Befallsflecken auf den Blättern ausgewertet.

In diesem Test zeigt die erfindungsgemäße Verbindung (1-2) eine wesentlich bessere Wirksamkeit als die Vergleichssubstanzen (A) und (B).

### Beispiel I

Sphaerotheca-Test (Gurke) / protektiv
- Lösungsmittel:: 4,7 Gewichtsteile Aceton
- Emulgator:: 0,3 Gewichtsteile Alkylarylpolyglykolether

Zur Herstellung einer zweckmäßigen Wirkstoffzubereitung vermischt man 1 Gewichtsteil Wirstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Sphaerotheca fuliginea bestäubt

Die Pflanzen werden anschließend bei 23 bis 24°C und bei einer relativen Luftfeuchtigkeit von ca 75 % im Gewächshaus aufgestellt.

10 Tage nach der Inokulation erfolgt die Auswertung.

In diesem Test zeigt die erfindungsgemäße Verbindung (I-2) eine wesentlich bessere Wirksamkeit als die Vergleichssubstanzen (A) und (B).

## Patentansprüche

1. Halogenalkyl-azolyl-Derivate der Formel 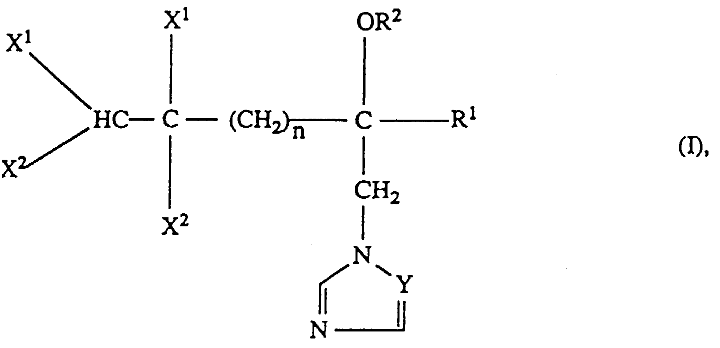 in welcher
R¹ für geradkettiges oder verzweigtes Alkyl mit 1 bis 6 Kohlenstoffatomen steht, wobei jeder dieser Reste einfach bis dreifach, gleichartig oder verschieden substituiert sein kann durch Halogen und/oder Cycloalkyl mit 3 bis 7 Kohlenstoffatomen, oder
R¹ für Cycloalkyl mit 3 bis 7 Kohlenstoffatomen steht, wobei jeder dieser Cycloalkylreste einfach bis dreifach, gleichartig oder verschieden substituiert sein kann durch Halogen und/oder Alkyl mit 1 bis 4 Kohlenstoffatomen,
R² für Wasserstoff, Alkyl mit 1 bis 6 Kohlenstoffatomen, Alkenyl mit 3 bis 6 Kohlenstoffatomen, Acyl mit 1 bis 4 Kohlenstoffatomen oder Phenylalkyl mit 1 bis 4 Kohlenstoffatomen im Alkylteil steht,
X¹ für Fluor, Chlor oder Brom steht,
X² für Fluor, Chlor oder Brom steht,
n für 0 oder 1 steht und
Y für Stickstoff oder eine CH-Gruppe steht,
sowie deren Säureadditions-Salze und Metallsalz-Komplexe.

2. Verfahren zur Herstellung von Halogenalkyl-azolyl-Derivaten der Formel (I) gemäß Anspruch 1 sowie von deren Säureadditions-Salzen und Metallsalz-Komplexen,
dadurch gekennzeichnet, daß man
Alkine der Formel 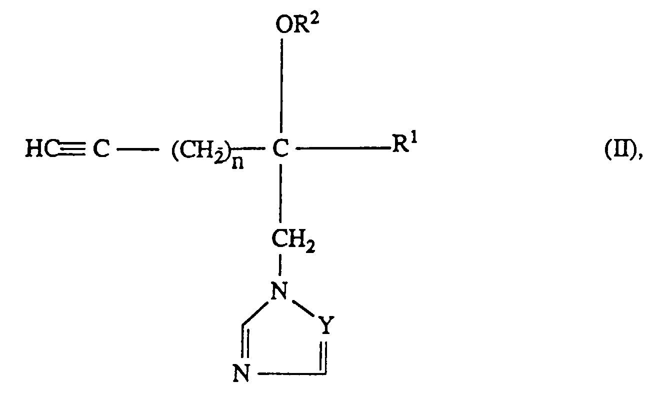 in welcher
R¹, R², Y und n die oben angegebenen Bedeutungen haben,
mit Halogen oder Halogen liefernden Verbindungen in Gegenwart eines Verdünnungsmittels umsetzt,
und gegebenenfalls anschließend an die so erhaltenen Verbindungen der Formel (I) eine Säure oder ein Metallsalz addiert.

3. Mikrobizide Mittel, gekennzeichnet durch einen Gehalt an mindestens einem Halogenalkyl-azolyl-Derivat der Formel (I) gemäß Anspruch 1 bzw. an einem Säureadditions-Salz oder Metallsalz-Komplex eines Halogenalkylazolyl-Derivates der Formel (I).

4. Verwendung von Halogenalkyl-azolyl-Derivaten der Formel (I) gemäß Anspruch 1 bzw. von deren Säureadditions-Salzen und Metallsalz-Komplexen als Mikrobizide im Pflanzenschutz und im Materialschutz.

5. Verfahren zur Bekämpfung von unerwünschten Mikroorganismen im Pflanzenschutz und im Materialschutz, dadurch gekennzeichnet, daß man Halogenalkyl-azolyl-Derivate der Formel (I) gemäß Anspruch 1 bzw. deren Säureadditions-Salze oder Metallsalz-Komplexe auf die Mikroorganismen und/oder ihren Lebensraum ausbringt.

6. Verfahren zur Herstellung von mikrobiziden Mitteln, dadurch gekennzeichnet, daß man Halogenalkyl-azolyl-Derivate der Formel (I) gemäß Anspruch 1 bzw. deren Säureadditionssalze oder Metallsalz-Komplexe mit Streckmitteln und/oder oberflächenaktiven Stoffen vermischt.

## Claims

1. Halogenoalkyl-azolyl derivatives of the formula 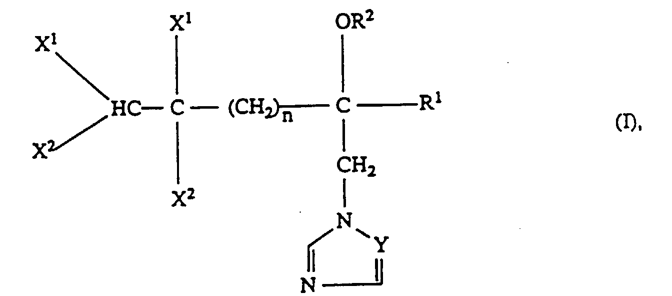 in which
R¹ represents straight-chain or branched alkyl having 1 to 6 carbon atoms, it being possible for each of these radicals to be monosubstituted to trisubstituted by identical or different substituents from the series compising halogen and/or cycloalkyl having 3 to 7 carbon atoms, or
R¹ represents cycloalkyl having 3 to 7 carbon atoms, it being possible for each of these cycloalkyl radicals to be monosubstituted or trisubstituted by identical or different substituents from the series comprising halogen and/or alkyl having 1 to 4 carbon atoms,
R² represents hydrogen, alkyl having 1 to 6 carbon atoms, alkenyl having 3 to 6 carbon atoms, acyl having 1 to 4 carbon atoms or phenylalkyl having 1 to 4 carbon atoms in the alkyl moiety,
X¹ represents fluorine, chlorine or bromine,
X² represents fluorine, chlorine or bromine,
n represents 0 or 1 and
y represnets nitrogen or a CH group
and their acid addition salts and metal salt complexes.

2. Process for the preparation of halogenoalkyl-azolyl derivatives of the formula (I) according to Claim 1 and their acid addition salts and metal salt complexes,
characterised in that
alkines of the formula 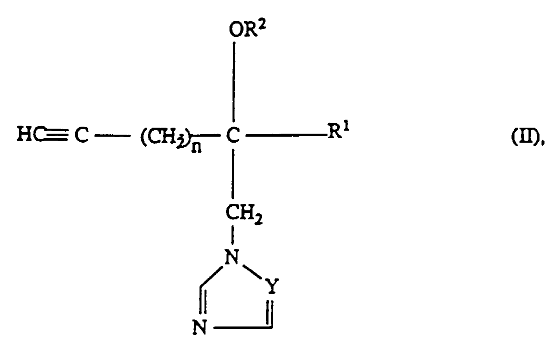 in which
R¹, R², Y and n have the abovementioned meanings,
are reacted with halogen or halogen-donating compounds in the presence of a diluent,
and, if desired, the resulting compounds of the formula (I) are subsequently subjected to an addition reaction with an acid or a metal salt.

3. Microbicidal agents, characterised in that they contain at least one halogenoalkyl-azolyl derivative of the formula (I) according to Claim 1, or an acid addition salt or metal salt complex of a halogenoalkyl-azolyl derivative of the formula (I).

4. Use of halogenoalkyl-azolyl derivatives of the formula (I) according to Claim 1 or of their acid addition salts and metal salt complexes as microbicides in plant protection and in the protection of materials.

5. Method of combating undesired microorganisms in plant protection and in the protection of materials, characterised in that halogenoalkyl-azolyl derivatives of the formula (I) according to Claim 1 or their acid addition salts or metal salt complexes are applied to the microorganisms and/or their habitat.

6. Process for the preparation of microbicidal agents, characterised in that halogenoalkyl-azolyl derivatives of the formula (I) according to Claim 1 or their acid addition salts or metal salt complexes are mixed with extenders and/or surface-active substances.

## Revendications

1. Dérivés d'halogénalkyl-azolyle répondant à la formule 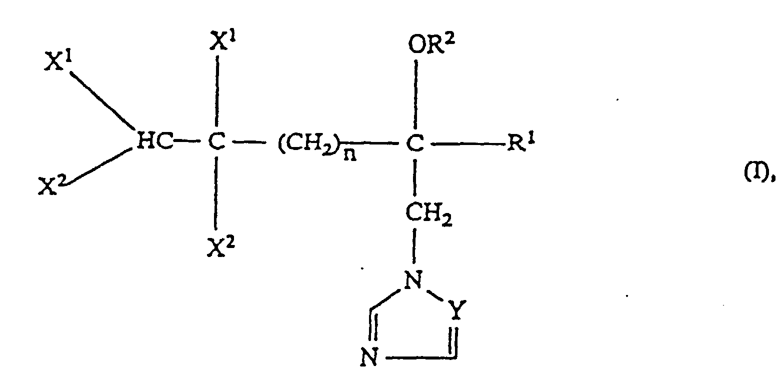 dans laquelle
R¹ représente un groupe alkyle à chaîne droite ou ramifiée contenant de 1 à 6 atomes de carbone, chacun de ces radicaux pouvant porter de 1 à 3 substituants identiques ou différents halogéno et/ou cycloalkyle contenant de 3 à 7 atomes de carbone, ou bien
R¹ représente un groupe cycloalkyle contenant de 3 à 7 atomes de carbone, chacun de ces radicaux cycloalkyle pouvant porter de 1 à 3 substituants identiques ou différents halogéno et/ou alkyle contenant de 1 à 4 atomes de carbone,
R² représente un atome d'hydrogène, un groupe alkyle contenant de 1 à 6 atomes de carbone, un groupe alcényle contenant de 3 à 6 atomes de carbone, un groupe acyle contenant de 1 à 4 atomes de carbone ou un groupe phénylalkyle contenant de 1 à 4 atomes de carbone dans la fraction alkyle,
X¹ représente un atome de fluor, un atome de chlore, un atome de brome,
X² représente un atome de fluor, un atome de chlore, un atome de brome,
n représente 0 ou 1, et
Y représente un atome d'azote ou un groupe CH,
ainsi que leurs sels d'addition d'acides et leurs complexes de sels métalliques.

2. Procédé pour la préparation de dérivés d'halogénalkyl-azolyle répondant à la formule (I) selon la revendication 1, ainsi que de leurs sels d'addition d'acides et de leurs complexes de sels métalliques,
caractérisé en ce qu'on fait réagir des alcynes de formule 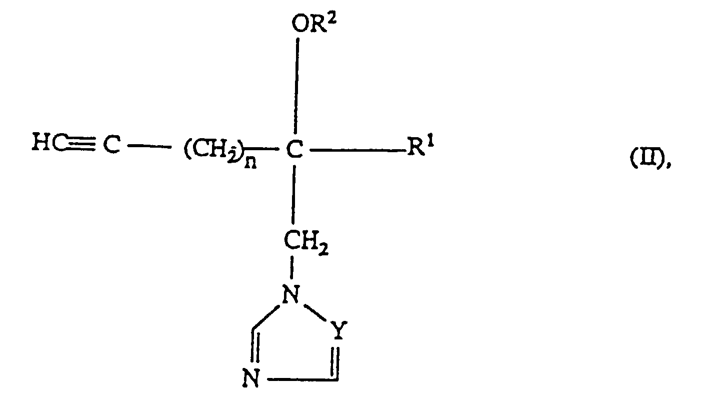 dans laquelle
R¹, R², Y et n on les significations indiquées ci-dessus,
avec un atome d'halogène ou avec des composés libérant des atomes d'halogène, en présence d'un diluant,
et éventuellement on ajoute ultérieurement aux composés de formule (I) ainsi obtenus, un acide ou un sel métallique.

3. Agents microbicides caractérisés par une teneur en au moins un dérivé d'halogénalkyl-azolyle de formule (I) selon la revendication 1, respectivement en un sel d'addition d'acide ou en un complexe de sel métallique d'un dérivé d'halogénalkyl-azolyle de formule (I).

4. Utilisation de dérivés d'halogénalkyl-azolyle de formule (I) selon la revendication 1, respectivement de leurs sels d'addition d'acides et de leurs complexes de sels métalliques, comme microbicides dans la protection des plantes et dans la protection des matériaux.

5. Procédé pour lutter contre des microorganismes indésirables dans la protection des plantes et dans la protection des matériaux, caractérisé en ce qu'on applique des dérivés d'halogénalkyl-azolyle de formule (I) selon la revendication 1, respectivement leurs sels d'addition d'acides ou leurs complexes de sels métalliques, sur les microorganismes et/ou sur leur biotope.

6. Procédé pour la préparation d'agents microbicides, caractérisé en ce qu'on mélange des dérivés d'halogénalkyl-azolyle de formule (I) selon la revendication 1, respectivement leurs sels d'addition d'acides ou leurs complexes de sels métalliques, avec des diluants et/ou des substances tensioactives.
